# EUROPÄISCHE PATENTANMELDUNG

(11) **EP 1 250 899 A1**
(43) Veröffentlichungstag der Anmeldung: **23.10.2002**
(21) Anmeldenummer: 01108544.6
(22) Anmeldetag: 05.04.2001
(51) Int. Cl.: A61F 6/08

(54) **Pessar**

(71) Anmelder: ProCon Gesellschaft für Kontinenzversorgung und Rehabilitation mbH, 22393 Hamburg (DE)
(72) Erfinder: Die Erfindernennung liegt noch nicht vor
(74) Vertreter: Vollmann, Heiko, Dipl.-Ing.

(57) **Zusammenfassung**

Das Pessar (1) weist mehrere nach außen offene Saugnäpfe (2) auf, wie dies bei Würfelpessaren üblich ist. Jeder Saugnapf (2) weist eine zentrale Ausnehmung (4) der Saugnapfwandung (3), die mittels eines Dichtkörpers (9) verschlossen ist. Zum Entfernen des Pessars kann der Dichtkörper aus seiner Verschließstellung in eine Haltestellung verbracht werden, in der die Saugnäpfe (2) über die dann geöffneten Ausnehmungen (4) druckausgeglichen sind, so dass das Pessar dann leicht entfernt werden kann.

## Beschreibung

Die Erfindung betrifft ein Pessar mit den im Oberbegriff des Anspruchs 1 angegebenen Merkmalen.

Die konservative Behandlung von Scheiden- oder Gebärmuttersenkungen mit Hilfe von Pessaren erfreut sich wieder zunehmender Beliebtheit in der Urogynäkologie. Erwartungen in neue operative Verfahren haben sich nur teilweise erfüllt, und insbesondere bei Operationen unter ambulanten Bedingungen sind erhebliche Nebenwirkungen bekannt geworden. Eine Vielzahl von Pessarformen ist zur Zeit im Einsatz, wie Produktinformationen der Hersteller zeigen, zum Beispiel von den Firmen Dr. Arabin GmbH oder Milex Incorporated. In der Praxis sind heute Siebschalenpessare. Rundringe und Würfelpessare die am meisten gebräuchlichen Typen, sie machen zusammen ca. 80 % der Anwendungen aus. Zu der wieder zunehmenden Verbreitung haben auch neue, gewebeverträglichere Werkstoffe, zum Beispiel Silikonpolymere, beigetragen.

Ein prinzipieller Nachteil der Ringe oder Schalen ist, dass sie einen zumindest noch eingeschränkt tragfähigen Beckenboden voraussetzen. Diese Bedingung ist bei älteren Patientinnen häufig nicht mehr gegeben. Bei diesen schwierigen Voraussetzungen werden heute meist Würfelpessare eingesetzt. Der therapeutische Effekt beruht auf dem Saugnapfeffekt der eingezogenen Würfelflächen. Der Würfel wird möglichst weit hinten in die Vagina eingeschoben und so positioniert, dass Gebärmutter und eventuell Vorfälle der Blase oder des Dickdarms in der Lage korrigiert werden. Die Flächen des Würfels haften sodann saugend an der Vaginalschleimhaut fest und unterstützen die Lage der Organe in korrigierter Position. Die starke Haftung der Würfel bedingt jedoch gleichzeitig einen gravierenden Nachteil beim Herausnehmen, der einer noch breiteren Verwendung entgegensteht.

Sexuell aktive Frauen akzeptieren Pessare nur dann, wenn Selbsteinlage und Wechsel einfach möglich sind. Auch bei nicht mehr sexuell aktiven älteren Frauen wäre das Herausnehmen während der Nacht wünschenswert, damit sich die Schleimhäute der Vagina erholen können und sich keine Drucknekrosen bilden. In der Realität gestaltet sich der Wechsel jedoch meist sehr schwierig, weil zum Lösen des Würfels beträchtliche Kräfte erforderlich sind. Die Würfel sind zum Herausziehen mit Griffbändern versehen, die entweder zentral oder entsprechend der Lehre der EP 0 636 355 B1 an einer Ecke des Würfels befestigt sind. Bei Pessaren gemäß EP 0 636 355 B1 soll eine diagonale Krafteinleitung entstehen, die zu einer drehenden Lösung der Saugflächen führen und damit das Wechseln erleichtern soll.

In der Praxis gelingt dies den Patienten jedoch meist nur mit schwierigen zusätzlichen Manipulationen. Die Hersteller empfehlen in ihren Gebrauchsanweisungen, die Saugflächen mit kreisenden Bewegungen der eingeschobenen Finger von der Vaginalschleimhaut zu lösen. Wenn die Patientinnen versuchen, die Würfel herauszuziehen, ohne vorab die Saugwirkung aufgelöst zu haben, kann dies zu einer erheblichen Verschlechterung der Prolapssituation beitragen, weil der Würfel dann wie eine Saugglocke die Gebärmutter mitzieht. Die Schmerzen bei fehlgeschlagenen Lösungsversuchen veranlassen viele Frauen, die Pessare bis zu Monaten ohne Reinigung in Position zu belassen.

Zur Vermeidung dieses Problems bieten die Hersteller seit einigen Jahren Würfelpessare mit gelochten Flächen an. In Abhängigkeit von Anzahl und Größe führen die Aussparungen zu einem deutlich geringeren Saugeffekt, womit der Wechsel erleichtert wird. Andererseits führt der reduzierte Saugeffekt dazu, dass die Pessare an Haltekraft verlieren und nicht sicher in ihrer Position verbleiben. Der Arzt setzt deshalb Modelle mit größeren Durchmessern ein, um die fehlende Saugwirkung mit einer Spreizhaftung auszugleichen. Diese führt jedoch wiederum zu einer mechanischen Reizung der Vaginalschleimhäute und wird von vielen Patientinnen wegen Druckschmerzen nicht dauerhaft toleriert.

Aus US-PS 4,669,478 und DE 198 16 349 sind Pessare bekannt, bei denen das Volumen variiert werden kann. Damit soll erreicht werden, dass die Pessare mit kleinem Volumen in die Vagina eingeführt und wieder heraus genommen werden können. Während der Tragephase werden diese Pessare mit Hilfe von angesetzten Schläuchen oder Kathetern mit Luft oder Flüssigkeit gefüllt. Alle diese Lösungen haben jedoch den Nachteil, dass die Schläuche ebenfalls in der Vagina verbleiben und dort zu störenden Reizungen oder Entzündungen führen können. In der Praxis hat sich deshalb keine der Lösungen als Produkt am Markt etablieren können.

Der Erfindung liegt die Aufgabe zugrunde, einen gattungsgemäßen Pessar mit mindestens einem nach außen offenen Saugnapf so auszubilden, dass er während des Tragens stark haftet, sich jedoch zum Zwecke des Wechselns oder des Herausnehmens schnell und einfach lösen lässt.

Diese Aufgabe wird gemäß der Erfindung durch die in Anspruch 1 angegebenen Merkmale gelöst. Vorteilhafte Ausgestaltungen der Erfindung sind in den Unteransprüchen, der nachfolgenden Beschreibung sowie der Zeichnung angegeben.

Grundgedanke der vorliegenden Erfindung ist es, zum Zwecke des Lösens des Pessars, insbesondere eines Saugnapfes des Pessars von der Vaginalwandung, durch eine Ausnehmung in der Saugnapfwandung einen Druckausgleich zu schaffen, um den Saugeffekt zu vermindern bzw. aufzuheben und das Pessar ohne Probleme entfernen zu können.

Eine solche Ausnehmung in einer Saugnapfwandung kann in Form einer Öffnung gebildet sein, aber auch erst zum Zwecke des Öffnens gebildet werden, wenn die Saugnapfwandung beispielsweise durch Wandungsabschnitte gebildet wird, die beim Öffnen einen Abstand zueinander aufweisen und dadurch eine Ausnehmung bilden. Zumindest die Mittel zum Öffnen sind vorteilhafterweise so angeordnet, dass sie in eingeführtem Zustand von außen zugänglich sind, beispielsweise durch einen Faden. Ist die Ausnehmung in der Saugnapfwandung hingegen verschlossen, so entsteht eine hohe Saugwirkung und damit auch eine gute Stützwirkung des Pessars.

Ein besonders einfacher Aufbau mit zuverlässiger und einfacher Handhabung des Pessars ergibt sich, wenn zum Verschließen der Ausnehmung ein Dichtkörper vorgesehen ist, der vorzugsweise rastend in der die Ausnehmung verschließenden Stellung liegt und mittels eines daran befestigten Fadens in eingeführtem Zustand des Pessars von außen aus dieser Stellung entfernbar ist. Ein solcher Dichtkörper kann beispielsweise eine kugelähnliche Form aufweisen, so dass er in einer Vielzahl von Stellungen dichtend die Ausnehmung verschließt, ohne hier gesondert positioniert zu werden. Eine solche Dichtungsanordnung hat darüber hinaus den Vorteil eines gewissen Selbstreinigungseffektes, so dass nicht die Gefahr besteht, dass sich im Bereich des Dichtsitzes Ablagerungen bilden.

Besonders vorteilhaft ist es, wenn das Pessar als Würfelpessar ausgebildet ist und die saugwirksamen Flächen des Würfels saugnapfartig ausgebildet sind. Dann kann in jeder dieser Flächen eine Ausnehmung vorgesehen sein, wobei bevorzugt sämtliche dieser Ausnehmungen durch einen gemeinsamen Dichtkörper, beispielsweise in Kugelform, verschlossen sind. In Arbeitslage sitzt dann dieser kugelförmige Dichtkörper zentral im Pessar, so dass die Saugnäpfe geschlossene Saugflächen aufweisen. Zum Entfernen des Pessars wird der Dichtkörper vorzugsweise mittels eines daran befestigten Fadens von außen aus seiner dichtenden Stellung entfernt, wodurch die Saugnäpfe druckausgeglichen und das Pessar aus der Körperhöhle herausgezogen werden kann.

Bevorzugt ist der Dichtkörper dabei so innerhalb des Pessars angeordnet, dass er in seiner die Ausnehmungen verschließenden Stellung eine Raststellung einnimmt und aus dieser Raststellung in eine proximale Haltestellung verbringbar ist, in welcher der Dichtkörper mit Spiel innerhalb des Pessars angeordnet ist, so dass ein Druckausgleich über die Ausnehmungen erfolgen kann. Vorteilhaft ist der Pessar so gestaltet, dass der Dichtkörper in dieser proximalen Haltestellung formschlüssig gehalten ist, so dass der Pessar über den Dichtkörper und den daran angebrachten Faden herausgezogen werden kann, ohne dass die Gefahr besteht, dass sich der Dichtkörper vom Pessar löst. Hierzu kann beispielsweise ein käfigartiges Gebilde am Pessar an- bzw. eingeformt sein, das die Luftzufuhr um den Dichtkörper herum zu den Ausnehmungen ermöglicht, gleichzeitig jedoch den Dichtkörper zuverlässig innerhalb des Pessars in proximaler Richtung festhält.

Um einerseits einen hohen Tragekomfort und andererseits eine gute Saugwirkung zu erzielen, ist es zweckmäßig, das Pessar aus einem die Saugnäpfe bildenden Grundkörper aus weichelastischem, körperverträglichen Polymer, vorzugsweise auf Silikonbasis, zu bilden, den darin befindlichen Dichtkörper hingegen aus einem vergleichsweise harten Polymer. Dann kann die Rastfunktion der Verschließstellung durch den Grundkörper selbst gebildet werden, indem der Durchgang zur Haltestellung entsprechend eng ausgebildet wird, so dass er nur durch Dehnung derselben mittels des Dichtkörpers durchdrungen werden kann.

Da das Pessar nicht nur durch die Saugnapffunktion haftet, sondern auch durch Flächenadhäsion, ist die Dimensionierung zwischen der den eigentlichen Saugnapf bildenden Fläche und der der Ausnehmung, die durch den Dichtkörper verschlossen wird, von Bedeutung. Wenn die Haftwirkung der Saugflächen trotz herausgezogenen Dichtkörpers nach wie vor so groß ist, dass das Pessar nicht oder nur schwer entfernt werden kann, dann kann ein Lösen durch manuelles Wiedereinschieben des Dichtkörpers in die Schließstellung erreicht werden. Bei diesem Vorgang wird gleichzeitig Luft in das Pessarinnere gepumpt und durch die in die Ausnehmungen eindrückende Kugel das Lösen der Vaginalschleimhaut vom Pessar unterstützt. Es hat sich gezeigt, dass für diesen Effekt die durch den Dichtkörper verschließbare Fläche der Ausnehmung, d. h. die in den Saugnapf eindringende Kugelfläche, etwa 20 % bis 40 % der Saugfläche, also der gesamten Fläche der Saugnapfwandung, betragen sollte.

Die Erfindung ist nachfolgend anhand eines in der Zeichnung dargestellten Ausführungsbeispiels erläutert. Es zeigen:
- Fig. 1: ein erfindungsgemäßes Pessar in vereinfachter perspektivischer Darstellung von schräg oben,
- Fig. 2: das Pessar gemäß Fig. 1 in perspektivischer Darstellung von schräg unten,
- Fig. 3: einen Querschnitt durch das Pessar und
- Fig. 4: einen Längsschnitt durch das Pessar längs der Schnittlinie IV-IV in Fig. 3.

Bei dem in den Figuren dargestellten Pessar 1 handelt es sich um ein Würfelpessar mit insgesamt fünf Saugnäpfen 2 im Wesentlichen gleicher Form mit Saugnapfwandungen 3, deren Saugflächen kegelstumpfartige Gebilde aufspannen. Jeder Saugnapf 2 ist mit einer im Wesentlichen kreisrunden Ausnehmung 4 versehen, die in einem zentralen Hohlraum 5 eines Grundkörpers 6 liegt, der auch die Saugnäpfe 2 bildet. Dieser zentrale Hohlraum 5 ist über einen Kanal 7 mit einem proximalseitig angeformten Käfig 8 verbunden, der ebenfalls Teil des Grundkörpers 6 und einstückig mit diesem ausgebildet ist.

Innerhalb des Grundkörpers 6 ist ein Dichtkörper 9 eingegliedert, der aus einer den eigentlichen Dichtkörper bildenden Kugel 10 sowie einer daran proximalseitig anschließenden Stange 11 besteht, nahe deren Ende ein Faden 12 befestigt ist.

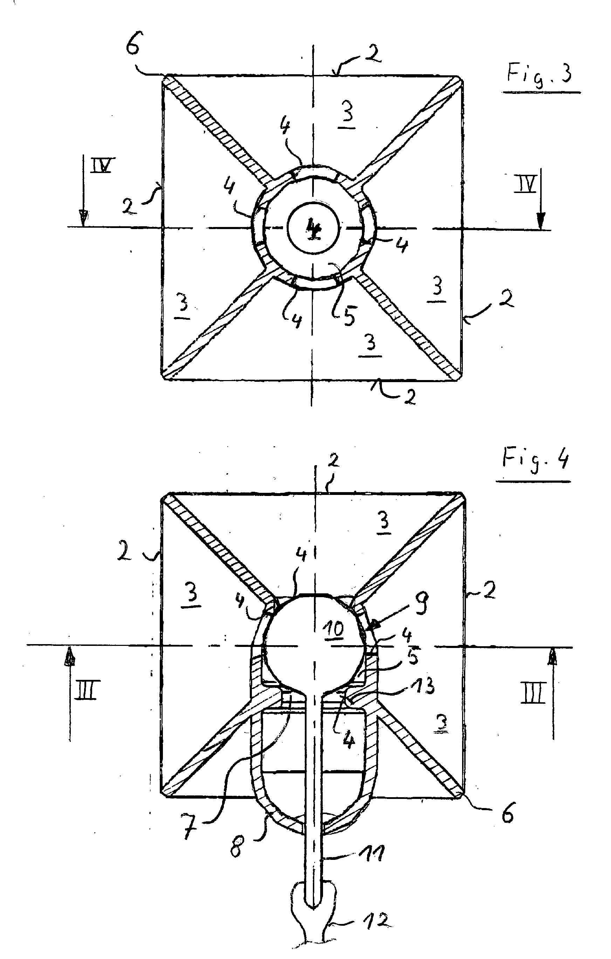

Wenn der Dichtkörper 9 in der in den Figuren, insbesondere in Fig. 4 dargestellten Stellung, welche die Verschließstellung bildet, angeordnet ist, dann werden durch die Kugel 10 alle fünf Ausnehmungen 4 der Saugnäpfe 2 dicht verschlossen. Der Dichtkörper 9 ist in dieser Verschließstellung rastend mittels eines Innenwulstes 13 gehalten, der den Kanal 7 umfangseitig begrenzt und einen kleineren Innendurchmesser als die Kugel 10 aufweist.

Da der Grundkörper 6 aus einem weichelastischen Polymer auf Silikonbasis gebildet ist, während der Dichtkörper 9 aus einem vergleichsweise harten Polymer besteht, kann durch proximalseitige Zugkrafteinleitung über den Faden 12 der Dichtkörper 9 aus seiner distalen Verschließstellung in eine proximale Haltestellung überführt werden. Dann gelangt die Kugel 10 unter Aufdehnung des Wulstes 13 in den Käfig 8, der fluchtend zum zentralen Hohlraum 5, proximalseitig zu diesem über den Kanal 7 verbunden, angeordnet ist. In dieser Stellung sind die Ausnehmungen 4 geöffnet, über den zentralen Hohlraum 5 und den Kanal 7 mit dem Käfig 8 und damit mit der proximalen Seite des Grundkörpers 6 leitungsverbunden, so dass die Saugnäpfe 2 druckausgeglichen sind.

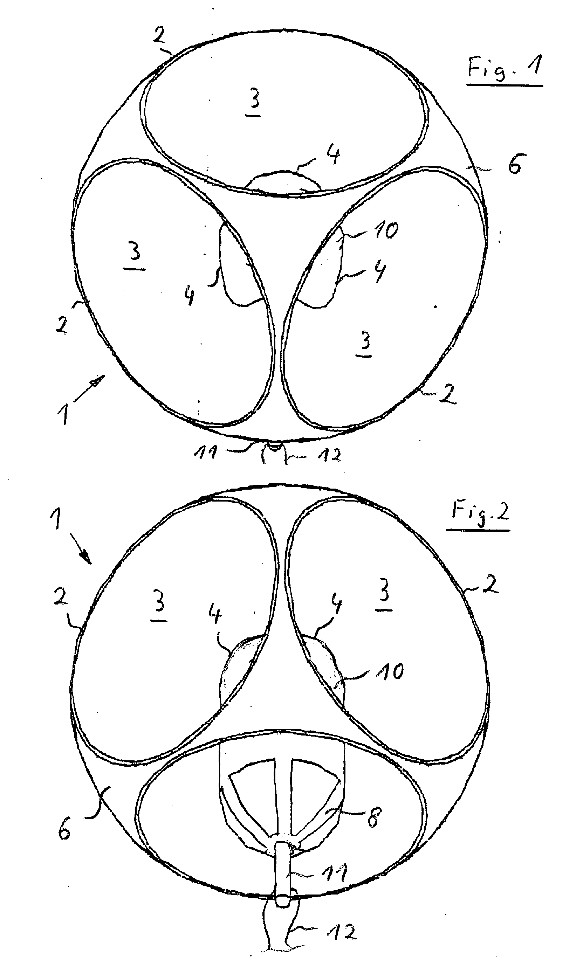

Die Anordnung und Ausbildung des Käfigs 8 ist, wie in Fig. 2 sichtbar, so gewählt, dass die Kugel 10 formschlüssig in proximaler Richtung gehalten wird. Das Einsetzen des Dichtkörpers 9 in den Grundkörper 6 erfolgt durch die distale Ausnehmung. Hierdurch kann der Dichtkörper 9 auch aus dem Grundkörper 6 entfernt werden, beispielsweise um beides gründlich reinigen zu können.

Die Saugnäpfe 2 sind zwar, wie die Fig. 3 und 4 verdeutlichen, auf den gedachten Flächen eines Würfels angeordnet, der Grundkörper 6 hat jedoch von seiner Außenkontur her eine Kugelform, wie sich auch aus den Fig. 1 und 2 ohne weiteres ergibt.

Das Pessar 1 wird mit dem in Verschließstellung (siehe Fig. 4) befindlichen Dichtkörper 9 in an sich bekannter Weise vaginal eingeführt und positioniert, wobei die Saugnäpfe 2 an der Vaginalschleimhaut anhaften, wodurch das Pessar 1 die gewünschte Stützwirkung erzielt.

Zum Entfernen des festgesaugten Pessars 1 wird zunächst über den Faden 12, der nach Art des Zugfadens eines Scheidentampons angeordnet ist, von außen eine nach proximalwärts gerichtete Zugkraft auf den Dichtkörper 9 aufgebracht, der hierdurch aus seiner Verschließstellung unter Überwindung der Haltekraft des sich dabei elastisch aufweitenden Wulstes 13 in seine proximale Haltestellung überführt wird, in der er innerhalb des Käfigs 8 mit Spiel angeordnet ist. Dadurch werden die Saugnäpfe 2 mit der proximalen Seite des Pessars 1 druckausgeglichen, so dass bei weiterem Zug die proximalwärts gerichtete Kraft über die Kugel 10 auf den Käfig 8 und damit den Grundkörper 6 ausgeübt wird, wodurch dieser üblicherweise aus der Vagina herausgezogen wird.

Wenn jedoch, was in Einzelfällen auftreten kann, die Adhösionswirkung zwischen den Saugflächen und der Vaginalschleimhaut so groß ist, dass ein Entfernen des Pessars 1 in der vorbeschriebenen Weise nicht ohne weiteres möglich ist, besteht die Möglichkeit, durch manuelles Distalwärtsdrücken der Stange 11 den Dichtkörper 9 wieder in die Verschließstellung zu verbringen. Hierbei wird die im zentralen Hohlraum 5 befindliche Luft, da der proximalseitige Zugang durch die am Wulst 13 anliegende Kugel 11 versperrt ist, in die Saugnäpfe 2 gepumpt. Gleichzeitig drücken die im Bereich der Ausnehmungen 4 befindlichen Kugelabschnitte auf die Vaginalschleimhaut und unterstützen ein Ablösen derselben von den Saugflächen (Saugnapfwandungen 3), wonach das Pessar soweit gelockert wird, dass dann ein Entfernen in jedem Falle leicht möglich ist.

Nach dem Reinigen des Pessars 1 wird der Dichtkörper 9 wieder in die in Fig. 4 dargestellte Verschließstellung verbracht, wonach das Pessar wieder wie eingangs beschrieben eingeführt und ggf. entfernt werden kann.

Bei dem vorstehend beschriebenen Pessar handelt es sich um ein Würfelpessar mit vier seitlichen und einer distalseitigen Saugfläche. Die Wahl von Anzahl und Anordnung der Saugflächen kann jedoch auch anders sein, beispielsweise mit drei oder fünf seitlichen Saugflächen. Auch können die Saugflächen anders verteilt angeordnet sein, die Form des Pessars ist dann entsprechend angepaßt.

### Bezugszeichenliste

- 1 -: Pessar
- 2 -: Saugnäpfe
- 3 -: Saugnapfwandungen
- 4 -: Ausnehmungen
- 5 -: zentraler Hohlraum
- 6 -: Grundkörper
- 7 -: Kanal
- 8 -: Käfig
- 9 -: Dichtkörper
- 10 -: Kugel
- 11 -: Stange
- 12 -: Faden
- 13 -: Wulst

## Patentansprüche

1. Pessar mit mindestens einem nach außen offenen Saugnapf (2), **dadurch gekennzeichnet, dass** der Saugnapf (2) eine Ausnehmung (4) in der Saugnapfwandung (3) aufweist und dass Mittel (9) zum Öffnen und Verschließen der Ausnehmung (4) vorgesehen sind.

2. Pessar nach Anspruch 1, **dadurch gekennzeichnet, dass** die Ausnehmung (2) in der Saugnapfwandung (4) mittels eines Dichtkörpers (9) verschlossen ist, der zur Aufhebung oder Verminderung des Saugeffekts aus seiner die Ausnehmung (2) verschließenden Stellung entfernbar ist.

3. Pessar nach einem der vorhergehenden Ansprüche, **dadurch gekennzeichnet, dass** der Dichtkörper (9) rastend in der die Ausnehmung (4) verschließenden Stellung liegt und mittels eines daran befestigten Fadens (12) in eingeführtem Zustand des Pessars (1) von außen aus dieser Stellung entfernbar ist.

4. Pessar nach einem der vorhergehenden Ansprüche, **dadurch gekennzeichnet, dass** der Dichtkörper (9) zumindest in dem Bereich, mit dem er in der Ausnehmung (4) der Saugnapfwandung (3) liegt, eine kugelartige Form hat.

5. Pessar nach einem der vorhergehenden Ansprüche, **dadurch gekennzeichnet, dass** mindestens zwei nach außen offene Saugnäpfe (2) mit Ausnehmungen (4) in der Saugnapfwandung (3) vorgesehen sind, die durch denselben Dichtkörper (9) verschlossen sind.

6. Pessar nach einem der vorhergehenden Ansprüche, **dadurch gekennzeichnet, dass** es sich um einen Würfelpessar (1) mit vorzugsweise fünf nach außen offenen Saugnäpfen (2) handelt, die jeweils eine Ausnehmung (4) in der Saugnapfwandung (3) aufweisen, wobei alle Ausnehmungen (4) durch denselben Dichtkörper (9) verschlossen sind.

7. Pessar nach einem der vorhergehenden Ansprüche, **dadurch gekennzeichnet, dass** es aus dem Dichtkörper (9) und einem diesen aufnehmenden Grundkörper (6) aufgebaut ist, und dass der Dichtkörper wahlweise in einer distalen Verschließstellung oder einer proximalen Haltestellung innerhalb des Grundkörpers (6) liegt, wobei der Dichtkörper (9) in der Verschließstellung die Ausnehmungen (4) in den Saugnapfwandungen (3) verschließt und in der Haltestellung diese freigibt.

8. Pessar nach einem der vorhergehenden Ansprüche, **dadurch gekennzeichnet, dass** der Grundkörper (6) an seiner proximalen Seite Haltemittel (8) für den Dichtkörper (9) aufweist.

9. Pessar nach einem der vorhergehenden Ansprüche, **dadurch gekennzeichnet, dass** der Grundkörper (6) aus einem weichelastischen, körperverträglichen Polymer, vorzugsweise auf Silikonbasis, und der Dichtkörper (9) aus einem vergleichsweise harten Polymer besteht.

10. Pessar nach einem der vorhergehenden Ansprüche, **dadurch gekennzeichnet, dass** die Haltemittel durch einen am Grundkörper (6) proximalseitig angeformten Käfig (8) gebildet sind, der den Dichtkörper (9) in der Haltestellung proximalseitig formschlüssig mit Spiel innerhalb des Grundkörpers (6) hält.
